(19) Europäisches Patentamt
European Patent Office
Office européen des brevets

(11) EP 0 793 499 B1

(12) EUROPEAN PATENT SPECIFICATION

(45) Date of publication and mention of the grant of the patent:
**19.10.2005 Bulletin 2005/42**

(21) Application number: **95942611.5**

(22) Date of filing: **12.12.1995**

(51) Int Cl.[7]: **A61K 31/555**, A61K 31/335, A61K 33/04, A61K 31/21, A61P 35/00

(86) International application number:
**PCT/US1995/016097**

(87) International publication number:
**WO 1996/018401 (20.06.1996 Gazette 1996/28)**

# (54) METHOD AND COMPOSITION FOR REDUCING TUMOR DEVELOPMENT WITH A COMBINATION OF A TAXANE COMPOUND AND A TELLURIUM AND/OR SELENIUM COMPOUND

VERFAHREN UND MITTEL ZUR VERMINDERUNG DER TUMORENTWICKLUNG MITTELS EINER KOMBINATION AUS EINER TAXANVERBINDUNG UND EINER TELLUR UND/ODER SELENVERBINDUNG

PROCEDE ET COMPOSITION PERMETTANT DE REDUIRE LE DEVELOPPEMENT D'UNE TUMEUR PAR ASSOCIATION D'UN COMPOSE DU TAXANE ET D'UN COMPOSE DU TELLURE ET/OU DU SELENIUM

(84) Designated Contracting States:
**AT BE CH DE DK ES FR GB GR IE IT LI LU MC NL PT SE**

(30) Priority: **15.12.1994 US 357128**

(43) Date of publication of application:
**10.09.1997 Bulletin 1997/37**

(73) Proprietor: **BAKER NORTON PHARMACEUTICALS, INC.**
**Miami, Florida 33178-2404 (US)**

(72) Inventors:
- **SREDNI, Benjamin**
  **Kfar-Saba (IL)**
- **BRUCKNER, Howard B.**
  **New York, NY 10024 (US)**
- **ALBECK, Michael**
  **Ramat-Gan (IL)**
- **WHISNANT, John**
  **Weston, FL 33332 (US)**
- **METTINGER, Karl L.**
  **Coral Gables, FL 33334-3516 (US)**

(74) Representative: **De Gregori, Antonella et al**
**Ing. Barzano' & Zanardo Milano S.p.A.**
**Via Borgonuovo 10**
**20121 Milano (IT)**

(56) References cited:

| | |
|---|---|
| **EP-A- 0 182 317** | **EP-A- 0 194 393** |
| **EP-A- 0 372 620** | **EP-A- 0 583 026** |
| **US-A- 4 761 490** | **US-A- 4 814 470** |
| **US-A- 4 876 399** | **US-A- 4 962 207** |

- **GUIGON M. ET AL: "Bone marrow protection." BONE MARROW TRANSPLANTATION, (1994) 13/1 (93-95). , XP000918425**
- **KALECHMAN, Y. ET AL: "Synergistic antitumor effect of the immunomodulator ASioi and taxol in experimental murine model." EXPERIMENTAL HEMATOLOGY (CHARLOTTESVILLE), (1995) VOL. 23, NO. 8, PP. 799. MEETING INFO.: 24TH ANNUAL MEETING OF THE INTERNATIONAL SOCIETY FOR EXPERIMENTAL HEMATOLOGY DUESSELDORF, GERMANY AUGUST 27-31, 1995 , XP000918420**
- **VADHAN-RAJ S. ET AL: "Phase I-II study of ossirene (IVX-Q-101 or AS-101) in patients (PTS) with ovarian cancer : reduction in chemotherapy (CT)-induced allopecia" PROC. ANNU. MEET. AM. SOC. CLIN. ONCOL., vol. 14, March 1995 (1995-03), page a712 XP000918561**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

## BACKGROUND OF THE INVENTION

### Field of the Invention:

This invention relates to a novel combination of chemotherapeutic agents which are useful in the treatment of neoplastic diseases.

The treatment is based on the sequential or concurrent administration of a taxane compound and a tellurium and/or a selenium compound in combination to provide an improved chemotherapeutic regimen for the treatment of neoplastic diseases or the treatment of malignant neoplasms. It has been discovered that the combination of a taxane compound and a tellurium and/or a selenium compound significantly reduces the development of tumor volume over what would be predicted from administration to tumor-infected mammals of either a taxane compound alone, or the tellurium and/or selenium compound, alone, or the tellurium compound in combination with cyclophosphamide.

In the prior art, paclitaxel and its precursors, isomers, analogs, isosteres, and derivatives (hereinafter "taxane compound(s)") have been described for the treatment of malignant neoplasms, such as acute leukemias and solid tumors. Moreover, ammonium trichloro(dioxoethylene-O,O-tellurate) (AS101) and its precursors, analogs, isosteres, and derivatives (hereinafter "tellurium and/or selenium compound(s)") have also been described for the treatment of such malignant neoplasms. It has also been suggested in the prior art to use tellurium compounds in combination with other chemotherapeutic agents for the treatment of such malignant neoplasms. In fact, it is known that a combination of ammonium trichloro(dioxoethylene-O,O-tellurate (AS101) and the known chemotherapeutic agent cyclophosphamide (CYP) shows synergism in extending the rate of survival of mice infected with Madison lung carcinoma tumor cells. Cancer Res. 51(5)1499-1503(1991).

It has now been discovered, and is the subject of the present invention, that a novel combination of the taxane compound paclitaxel and a tellurium and/or a selenium compound shows an unexpectedly strong synergistic effect on the reduction in tumor volume in mice infected with cells of the solid tumor B16 Melanoma. Such findings are unexpected because the use of either the taxane compound alone or the tellurium and/or selenium compound alone at the same respective dosages produce tumor volume reductions which are shorter lasting and incrementally much closer to the saline control.

The present invention is concerned with a novel treatment regimen which combines a taxane compound and a tellurium and/or selenium compound. It also contemplates a novel composition with anti-neoplastic activity comprising a combination of a taxane compound and a tellurium and/or selenium compound. Among the preferred features of the invention are regimens and novel therapeutic combinations wherein the ratios of the taxane compound and tellurium and/or selenium compound provide synergistic activity, especially in the reduction of solid tumor growth.

It is therefore an object of the invention to provide novel therapeutic regimens and compositions which are based on the sequential or concurrent administration of novel combination of a taxane compound and a tellurium and/or selenium compound to mammals infected with neoplasms.

It is also an object of this invention to provide a novel method for the treatment of neoplastic diseases which uses a particular dose of a novel combination of a taxane compound and a tellurium and/or selenium compound.

These and other objects of the invention will become apparent from a review of the specification.

### Brief Description of the Drawing

The effect of administration of the saline control, the individual components and the combination of paclitaxel and the tellurium compound, ammonium trichloro(dioxoethylene-O,O-tellurate) (AS101) on the development of solid B16 tumor in vivo over a 25 day period in infected mice are presented in graphical form in the Drawing. The bottom set of data points represent the present invention. It can be seen that tumor volume with the instant combination remains unexpectedly constant for the full 25 days.

### Summary of the Invention

In accordance with the present invention there is provided a method of treating malignancies which are sensitive to the combination of a taxane compound, and a tellurium and/or selenium compound, said method comprising administering, sequentially or concurrently, an amount of the combination which is effective to treat malignancies which are sensitive to the combination. Preferred features of the invention comprise administering the combination in weight ratios which are synergistically-effective to treat malignancies, especially solid tumors. Paclitaxel is preferred but other taxane compounds, such as the family of compounds known as taxoteres, can be used. Ammonium trichloro(dioxoethylene-O,O-tellurate) (AS101) is preferred but other tellurium and/or selenium compounds, such as dichlorodioxoethylene-O,O-tellurate, and the corresponding selenium isosteres, may be employed.

**Detailed Description of the Invention**

Paclitaxel is a member of the taxane family of diterpenes. Paclitaxel and analogs, derivatives, and compounds useful in the present invention generally will have the structure shown below:

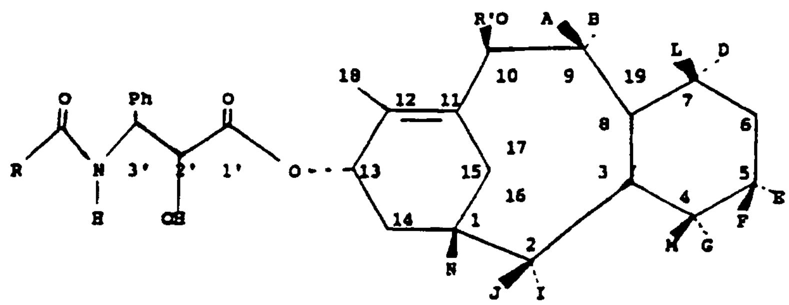

Paclitaxel itself is a compound of the above structure wherein A and B together form an oxo; L and N are hydroxy, D, E and J are hydrogen, G and R' are acetate; M and F together form an oxocyclobutyl ring; I is benzoyloxy; and R is phenyl.

The compound paclitaxel was reported by Wani et al. in JACS, 93, 2325, (1971) to exhibit significant anti-tumor properties. More specifically, paclitaxel shows strong cytotoxicity in KB cell structures and in several of the National Cancer Institute's in vivo screens, including P-388, L-1210 and P-1534 mouse leukemias, the B-16 melanocarcinoma, the CX-1 colon xenograft, the LX-1 lung xenograft and the MX-1 breast xenograft. In addition, paclitaxel blocks cell replication in HeLa cells, especially in the mitotic phase of cell division. More specifically, it has been reported by Schiff et al in Nature, 277, 665 (1979) that paclitaxel promotes the assembly of microtubule proteins responsible for the formation of spindles during cell division.

Because of its promising anti-cancer activity and its unusual structure and mechanism of action, paclitaxel represents a prototype of a new class of chemotherapeutic agents.

Members of the class are set forth in U.S. 4,876,399 and 4,814,470, and elsewhere. Preferred are those of the above structural formula wherein A and B are independently hydrogen or lower alkanoyloxy or A and B together form an oxo; L and D are independently hydrogen or hydroxy; E and F are independently hydrogen or lower alkanoyloxy or; E and F together form a oxo; G and R' are hydrogen or lower alkanoxy or lower alkanoyloxy or aroyloxy or G and M together form an oxo or methylene or G and M together form an oxocyclopropyl ring or M and F together form an oxocyclobutyl ring; J is hydrogen or aroyloxy or lower alkanoyloxy or I is hydrogen or aroyloxy; or I and J taken together form an oxo; N is hydrogen, hydroxy or lower alkoxy; and R is aryl, lower alkyl, lower alkoxy or lower alkenyl. The hydroxyl and the carbonylamido substituents attached to carbons designated 2' and 3' in the formula may be transposed without departing from the preferred family of compounds. The alkyl groups, either alone or with the various substituents defined hereinabove are preferably lower alkyl containing from one to about six carbon atoms in the principal chain and up to 10 carbon atoms. They may be straight or branched chain and include methyl, ethyl, propyl, isopropyl, butyl, isobutyl, tert-butyl, amyl and hexyl. The lower alkoxy groups have the same carbon atom contents as above and include methoxy, ethoxy, propoxy, isopropoxy, butoxy, isobutoxy, tert-butoxy, amyloxy and hexyloxy. Exemplary alkanoyloxy groups include acetate, propionate, butyrate, valarate and isobutyrate. The more preferred alkanoyloxy is acetate.

The aryl groups described hereinabove either alone or with various substituents contain from 6 to 10 carbon atoms and include phenyl, alpha-naphthyl or beta-naphthyl, etc. Phenyl is the more preferred aryl.

The lower alkenyl groups contain from 2 to 6 carbon atoms in the principal chain and up to a total of 10 carbon atoms. They may be straight or branched chain and include ethenyl, 1-propenyl, isopropenyl, 1-butenyl, 2-butenyl, 3-butenyl and isobutenyl.

Preferred values of the substituents A, B, L, D, G, F, G, M, I, J, and N are enumerated herein below:

A is hydrogen; B is hydrogen, or acetate or
A and B together from an oxo; L is hydrogen or hydroxy; D is hydrogen or hydroxy; E is hydrogen, or acetate or E and F together form an oxo or M and F together form an oxocyclobutyl ring; G is hydrogen or acetate or G and M together form an oxo or methylene or oxocyclopropyl ring; J is hydrogen; I is hydroxy or aroyloxy, such as

benzoyloxy or I and J together form an oxo; N is hydrogen, hydroxy or lower alkanoyloxy; R is phenyl or tert-butoxy; and R' is hydrogen or acetate.

All of the above-mentioned compounds, including paclitaxel itself can be made by methods set forth in the above-mentioned U.S. 4,876,399. Paclitaxel is commercially available as Taxol®

In addition, as set forth in U.S. 4,814,470, because of the difficulty of extracting paclitaxel from trunk barks of different species of Taxus (yew), the preparation of derivatives similar to paclitaxel from 10-deacetylbaccatin, which can be extracted relatively easily from yew leaves, has been proposed. The mentioned patent describes derivatives which demonstrate activity which is higher than that of paclitaxel. Such derivatives can also be used in the present invention. They differ primarily in that they contain tert-butoxycarbonylamido groups instead of benzoylamido groups on the ring and in the side chains. They are embraced by the above formula and are known as taxotenes.

The ability of organic derivatives of tellurium and selenium type of the following general formulae (A) - (F) to protect mice from the adverse effects of the anti-neoplastic alkylating agent cyclophosphamide (CYP), has been demonstrated [[Cancer Res. 51(5)1499-1503(1991)], and see U.S. Patent 4,761,490.

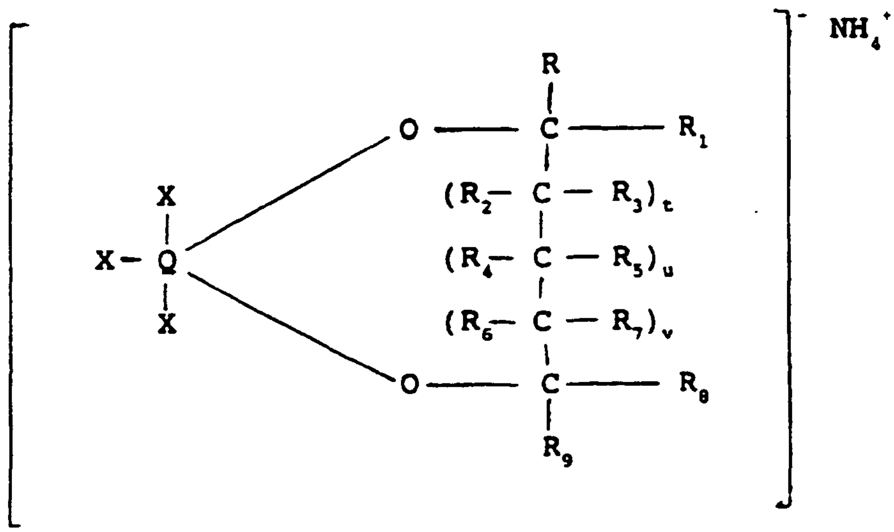

(A)

or

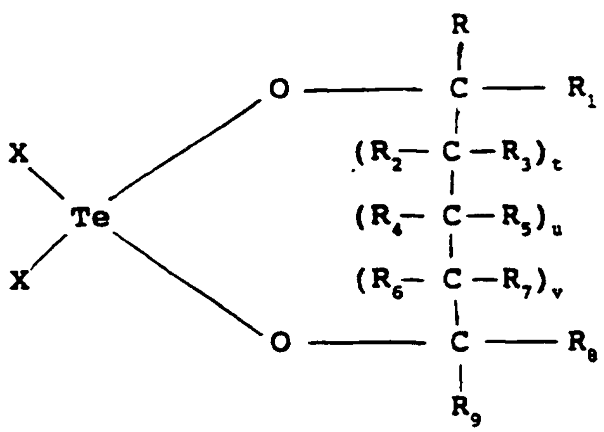

(B)

or

$$TeX_4 \qquad (C)$$

or

$$TeO_2 \quad (D)$$

or

$$PhTeCl_3 \quad (E)$$

or

$$(C_6H_5)\ ^{+}P(TeCl_3(O_2C_2H_4))^{-} \quad (F)$$

wherein Q is Te or Se; t is 1 or 0; u is 1 or 0; v is 1 or 0; R, $R_1$, $R_2$, $R_3$, $R_4$, $R_5$, $R_6$, $R_7$, $R_8$ and $R_9$ are the same or different and are independently selected from hydrogen, hydroxyalkyl of 1 to 6 carbons, hydroxy, alkyl of from 1 to 6 carbon atoms, halogen, haloalkyl of 1 to 6 carbon atoms, carboxy, alkylcarbonylalkyl of 2 to 10 carbons, alkanoyloxy of 1 to 6 carbon atoms, carboxyalkyl of 1 to 6 carbon atoms, acyl, amido, cyano, amidoalkyl of 1 to 6 carbons, N-monoalkylamidoalkyl of 2 to 10 carbons, N,N-dialkylamidoalkyl of 4 to 10 carbons, cyanoalkyl of 1 to 6 carbons, alkoxy of 1 to 6 carbon atoms, alkoxyalkyl of 2 to 10 carbon atoms and $-COR_{10}$ wherein $R_{10}$ is alkyl of from 1 to 6 carbons; and X is halogen. Although the ammonium salt is illustrated, it is understood that other pharmaceutically acceptable salts are within the scope of the invention. The compounds with the five membered rings are preferred.

As used herein, for compounds of formulae (A) and (B), the term alkyl of 1 to 6 carbon atoms includes straight and branched chain alkyl groups such as methyl; ethyl; n-propyl; and n-butyl; the term hydroxyalkyl of 1 to 6 carbon atoms includes hydroxymethyl; hydroxyethyl; hydroxy-n-butyl; the term haloalkyl of 1 to 6 carbon atoms includes chloromethyl; 2-iodoethyl; 4-bromo-n-butyle; iodoethyl; 4-bromo-n-pentyl; the term alkanoyloxy of 1 to 6 carbon atoms includes acetyl, propionyl, butanoyl; the term carboxyalkyl includes carboxymethyl, carboxyethyl and ethylenecarboxy; the term alkylcarbonylalkyl includes methanoylmethyl and ethanoylethyl; the term amidoalkyl includes $-CH_2CONH_2$; $-CH_2CH_2CONH_2$; $-CH_2CH_2CH_2CONH_2$ and the like; the term cyanoalkyl includes $-CH_2CN$; $-CH_2CH_2CN$; $-CH_2CH_2CH_2CN$; the term alkoxy of 1 to 6 carbon atoms includes methoxy, ethoxy, n-propoxy, n-pentoxy; the terms halo and halogen are used to signify chloro, bromo, iodo and fluoro; the term acyl includes $R_{16}CO$ wherein $R_{16}$ is H, or alkyl of 1 to 6 carbons such as methanoyl, ethanoyl; the term aryl includes phenyl, alkylphenyl and naphthyl; the term N-monoalkylamidoalkyl includes $-CH_2CH_2CONHCH_3$, $-CH_2CONHCH_2CH_3$; the term N,N-dialkylamidoalkyl includes $-CH_2CON(CH_3)_2$; $CH_2CH_2COM(CH_2CH_3)$. Compounds which are based on tellurium are the presently preferred compounds of the invention.

Ammonium (trichloro(dioxoethylene-O,O-tellurate) is a compound of the structure

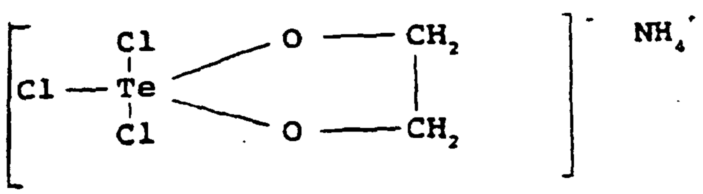

This compound is a member of a family of organic 9,10,11 derivatives of tellurium and selenium found to have the ability to stimulate the in vivo and in vitro production of cytokines and their receptors. As is set forth in U.S. Patent No. 4,761,490, these compounds may be utilized in the treatment of certain tumor, autoimmune diseases, immune diseases and infectious diseases.

The derivatives of tellurium or selenium that are useful in the present invention include those compounds of the above mentioned general formulas (A) - (F) which stimulate cells to produce lymphokines.

The compounds are made by combining substantially equimolar amounts of the reactants in a suitable reactor at room temperature or at elevated temperatures up to the reflux temperature. It is preferred to utilize a solvent that is capable of dissolving the reactants such as acetonitrile, benzene, toluene, xylene, dimethylsulfoxide, mixtures thereof and the like. Compounds of structure (A) are only obtained in acetonitrile. The preferred method requires heating the reaction mixture to the reflux temperature of the solvent while stirring the reaction mixture with a suitable magnetic or

mechanical stirrer. The reaction may be carried out for a sufficient period of time to ensure complete reaction of the reactants. This time will vary with the reaction conditions, the particular compound being made and the nature of the solvents. The reaction may be run at atmospheric pressure but if desired may be carried out at reduced or elevated pressure. The reaction is practically carried out in the presence of an oxygen containing atmosphere such as air but inert atmospheres such as nitrogen, argon, helium or mixtures thereof may be utilized if desired. Reaction times of 1 minute to 168 hours may be used although reaction times of 6-16 hours are preferred.

The drugs may be given simultaneously or separately. It is preferred to administer the tellurium or selenium compound prior to or simultaneously with the taxane compound. When the drugs are administered in the same composition, the composition will have effective amounts of both drugs to be administered by the same route of administration which will be determined from the therapeutic regimen which is adopted for an individual patient to provide more than an additive effect which could be expected from the use of either drug alone. For example a single dose vial may be prepared which combines 30mg of paclitaxel and 3mg of ammonium (trichloro(dioxoethylene-O,O-tellurate) in an appropriate vehicle such as the vehicle which is commercially used for Taxol®.

It has been found that a preferred two component therapeutic regimen of the invention is based on the use of the following dosages of the two drugs:

(a) a taxane compound is administered at a dose of 130mg per $M^2$ to 210mg per $M^2$ given by intravenous infusion once every 20 to 40 days, as necessary.

(b) the tellurium compound may be administered by the oral, intramuscular, intravenous, transdermal or intraperitoneal route. The oral dose will be 0.15 to 0.5 mg/kg of body weight daily and preferably from 0.03 to 0.3 mg/kg of body weight daily in one dose or in divided doses. The parenteral dose will be 0.03 to 0.2 mg/kg of body weight daily and preferably from 0.006 to 0.02 mg/kg daily given as a bolus injection or as a continuous parenteral infusion.

The course of therapy may be carried out for a period of 2-52 weeks or longer. Depending on the therapeutic response and the manifestation of unacceptable side effects, the dose may be increased or decreased within the general description set forth herein.

The efficacy of the invention has been verified in a mouse model by a dosage regimen which begins three days after tumor implantation and uses 10 micrograms of tellurium or selenium compound/mouse IP every other day and to use 4 IP injections of paclitaxel at 17 mg/kg every other day starting on day 4 and continuing on days 6,8 and 10. The volume of the tumors is monitored (length X width) on a daily basis.

The drugs are preferably administered parenterally as a solution of the drug in normal or phosphate-buffered saline.

The invention may be used to treat benign tumor and malignancies which are sensitive to the described treatment regimen. The malignancies may be primary or metastatic such as solid tumors, leukemias or lymphomas. These malignancies include colorectal carcinoma, espohageal carcinoma, stomach carcinoma, pancreatic carcinoma, liver carcinoma, small bowel carcinoma, lung tumors, CML, mesotheiloma, melanomas, biliary carcinoma, breast carcinoma and adenocarcinoma of unknown origin.

The effectiveness of the combination of the present invention have been demonstrated against the B16 melanoma cell line.

The compound ammonium trichloro(dioxoethylene-O,O-tellurate) (AS101) was prepared according to the methods of Albeck and Sredni, U.S. Patent No. 4,761,490, Example 1. In this procedure, ethylene glycol and tellurium tetrachloride are refluxed in acetonitrile and, after cooling, the product precipitates from the reaction mixture as a white precipitate.

The tellurium compound may be diluted in a phosphate-buffered saline at pH 7.4 and maintained at 4°C. Before use, it may be diluted to the desired concentration in phosphate-buffered saline.

Paclitaxel is obtained by macerating ground non-dried leaves of Taxus baccata L, in accordance with known methods. See, for example, Colin, Guenard, Gueritte-Voegelein, and Potier, U.S. 4,814,470 and the 1994 PDR pp670-672 which are incorporated by reference. Paclitaxel is chemically known as 5β,20-Epoxy-1,2α,4,7β, 10β, 13α-hexahydroxytax-11-en-9-one 4,10 diacetate 2-benzoate 13-ester with (2R, 3S)-N-benzoyl-3-phenylisoserine.

The paclitaxel powder (6mg/ml) is suspended in 527mg of Cremophor®EL and 49.7%v/v of dehydrated alcohol. Other formulations of the taxane compounds may be prepared as required for specific applications.

## DESCRIPTION OF THE PREFERRED EMBODIMENTS

The following examples are given to illustrate the invention and it is understood that they do not limit the scope of the invention.

EXAMPLE 1

This example demonstrates the anti-neoplastic activity of ammonium trichloro(dioxoethylene-O,O-tellurate) (AS101) in combination with paclitaxel.

C57/B1 mice were injected S.C. with 400,000 B16 Melanoma cells. Three days after tumor implantation, mice were divided into four groups of 8 mice each. One of the groups of mice (control) was injected with Saline every other day. The second group of mice (AS101) was injected with 10 micrograms of AS101/mouse I.P. every other day. The third group (paclitaxel) received 4 I.P. injections of 17 mg/kg of paclitaxel every other day starting on day 4. The last group (AS101+paclitaxel) received both treatments. Groups 2 and 4 received AS101 injections every other day until the end of the experiment.

Experimental Protocol

Day 0 Tumor implantation
Group 1 - Day 3, Saline every other day until the end of the experiment
Group 2 - Day 3, AS101, every other day until the end of the experiment.
Group 3 - Day 4,6,8,10, paclitaxel
Group 4 - Day 3, AS101, every other day until the end of the experiment; Days 4,6,8,10, paclitaxel.

Every day the volume of the tumors was monitored (length X width).The results of the experiments are set forth as a graph in the Drawing.

It can be seen that tumor growth is reduced with ammonium trichloro(dioxoethylene-O,O-tellurate) (AS101) and paclitaxel alone in comparison with the control, but that tumor growth is completely inhibited when AS 101 is administered in combination with paclitaxel in accordance with the present invention.

The data establishes that the combined use of the organotellurium compound (AS101) and paclitaxel provides a substantial increase in the cytotoxicity exhibited by either compound alone. Moreover the increase is of such magnitude that it is more than a merely additive effect and therefore is unexpected. This increase in anti-neoplastic activity allows the clinician to achieve a therapeutic effect which is much greater than would be expected from the use of organotellurium compound AS101 in combination with other chemotherapeutic agents known in the prior art, specifically, the combination of AS101 and cyclophosphamide, which merely extended the lives of the test subjects, but had no reported effect on the size of the tumors.

EXAMPLE 2

If the procedure of Example 1 is repeated, substituting, respectively, for the paclitaxel and the ammonium trichloro(dioxoethylene-O,O-tellurate) equivalent weight amounts of a taxane compound of the formula

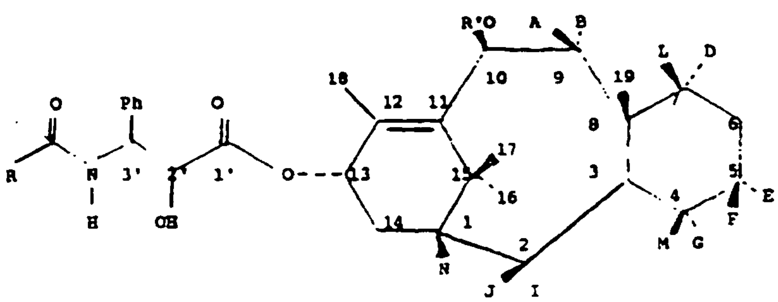

wherein R is tert-butyloxy, and R' is hydrogen (U.S. 4,814,470 (Example 2)) and an organotellurium compound of formula (B) above wherein X is Cl, and t, u, and v are 0 (U.S. 4,761,490) (Example 2), a composition in accordance with the present invention will be obtained.

EXAMPLE 3

If the procedure of Example 1 is repeated, substituting, respectively, for the paclitaxel and the ammonium

trichloro(dioxoethylene-O,O-tellurate) equivalent weight amounts of a taxol compound of the formula in Example 2 wherein R' is acetoxy, and R is tert-butyloxy, and an organotellurium compound of formula (A) wherein R is methyl and t, u, and v are 0 (U.S. 4,761,490) (Example 16), a composition in accordance with the instant invention will be obtained.

The present invention also provides pharmaceutical compositions containing a combination of a taxane compound and a tellurium or selenium compound as defined above, in further combination with one or more pharmaceutically acceptable, inert or physiologically active, diluents or adjuvants. These compositions may be presented in any form appropriate for the administration route envisaged. The parenteral route, and especially the intravenous route, is the preferred route for administration.

The compositions according to the invention for parenteral administration may be aqueous or nonaqueous sterile solutions, suspensions or emulsions. Propylene glycol, vegetable oils, especially olive oil, and injectable organic esters, e.g. ethyl oleate, may be used as the solvent or the vehicle. These compositions may also contain adjuvants, especially wetting agents, emulsifiers or dispersants. The sterilization may be carried out in several ways, e.g. using a bacteriological filter, by incorporating sterilizing agents into the composition, by irradiation or by heating. They may also be in the form of sterile solid compositions which may be dissolved or dispersed in sterile water or any other injectable sterile medium.

Many variations of the present invention will suggest themselves to those skilled in this art in light of the above, detailed description. For example, instead of a tellurium compound, the corresponding selenium compound can be used.

## Claims

1. A composition comprising a combination of a taxane compound and a tellurium or selenium compound in respective amounts effective to treat malignancies so as to produce more than a merely additive increase in therapeutic effect when compared to the taxane compound and the tellurium or selenium compound used alone, said tellurium or selenium compound being selected from the formulae:

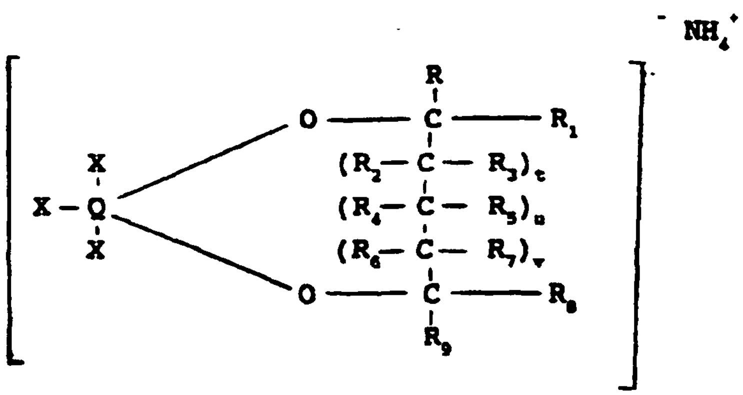

(A)

or

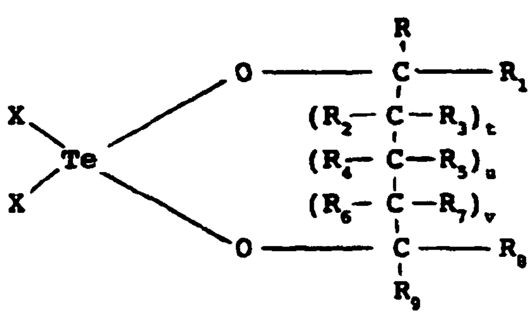

(B)

or

$$TeX_4 \quad (C)$$

or

$$TeO_2 \quad (D)$$

or

$$PhTeCl_3 \quad (E)$$

or

$$(C_6H_5)\ ^{+}P(TeCl_3(O_2C_2H_4))^{-} \quad (F)$$

wherein Q is Te or Se; t is 1 or 0; u is 1 or 0; v is 1 or 0; R, $R_1$, $R_2$, $R_3$, $R_4$, $R_5$, $R_6$, $R_7$, $R_8$ and $R_9$ are the same or different and are independently selected from hydrogen, hydroxyalkyl of 1 to 6 carbons, hydroxy, alkyl of from 1 to 6 carbon atoms, halogen, haloalkyl of 1 to 6 carbon atoms, carboxy, alkylcarbonylalkyl of 2 to 10 carbons, alkanoyloxy of 1 to 6 carbon atoms, carboxyalkyl of 1 to 6 carbon atoms, acyl, amido, cyano, amidoalkyl of 1 to 6 carbons, N-monoalkylamidoalkyl of 2 to 10 carbons, N,N-dialkylamidoalkyl of 4 to 10 carbons, cyanoalkyl of 1 to 6 carbons, alkoxy of 1 to 6 carbon atoms, alkoxyalkyl of 2 to 10 carbon atoms and $-COR_{10}$ wherein $R_{10}$ is alkyl of from 1 to 6 carbons; and X is halogen.

2. A composition as defined in Claim 1 wherein the taxane compound is selected from those of the formula:

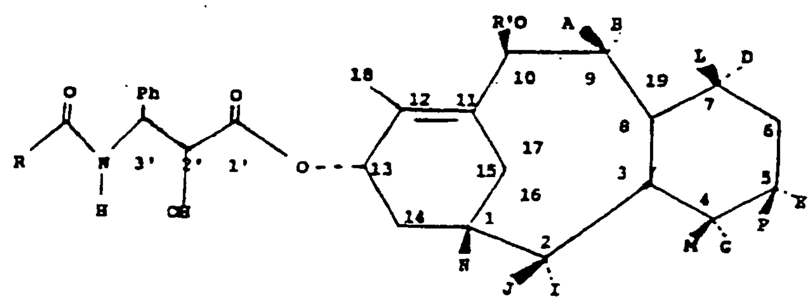

wherein

A and B are independently hydrogen or alkanoyloxy or
A and B together form an oxo;
L and D are independently hydrogen or hydroxy;
E and F are independently hydrogen or alkanoyloxy of from 1 to 6 carbon atoms or;
E and F together form an oxo;
G and R' are hydrogen or alkanoxy of from 1 to 6 carbon atoms or alkanoyloxy of from 1 to 6 carbon atoms or aroyloxy or
G and M together form an oxo or methylene or
G and M together form an oxocyclopropyl ring or
M and F together form an oxocyclobutyl ring;
J is hydrogen or aroyloxy or alkanoyloxy of from 1 to 6 carbon atoms or
I is hydrogen or aroyloxy; or
I and J taken together form an oxo;
N is hydrogen, hydroxy or alkoxy of from 1 to 6 carbon atoms; and
R is aryl, alkyl of from 1 to 6 carbon atoms, alkoxy of from 1 to 6 carbon atoms or alkenyl of from 2 to 6 carbon atoms;

and the hydroxyl and the carbonylamido substituents attached to carbons designated 2' and 3' in the formula may

be transposed.

3. A composition as defined in Claim 2 wherein the taxane compound is paclitaxel, a compound of the said formula wherein A and B together form an oxo; L and N are hydroxy, D, E and J are hydrogen, G is acetate; M and F together form an oxocyclobutyl ring; I is benzoyloxy; and R is phenyl.

4. A composition as defined in Claim 1 wherein the tellurium or selenium compound is ammonium trichloro(dioxoethylene-O,O-tellurate), a compound of formula (A) wherein Q is tellurium; R, $R_1$, $R_8$ and $R_9$ are hydrogen; and t, u, and v are 0.

5. A composition as defined in Claim 1 wherein the taxane compound comprises from about 10 to about 90 parts by weight and the tellurium or selenium compound comprises from about 90 to about 10 parts by weight of the combination.

6. A pharmaceutical composition which contains a taxane compound and a tellurium or selenium compound, further combined with one or more pharmaceutically acceptable, inert or physiologically active diluents or adjuvants.

**Patentansprüche**

1. Zusammensetzung enthaltend eine Kombination einer Taxan-Verbindung und einer Tellur- oder Selen-Verbindung in entsprechenden, zum Behandeln von Bösartigkeiten wirksamen Mengen, um mehr als nur eine additive Zunahme in der therapeutischen Wirkung zu erzielen, wenn mit der Taxan-Verbindung und der Tellur- oder Selen-Verbindung alleine eingesetzt verglichen, wobei die Tellur- oder Selen-Verbindung ausgewählt ist aus der Formel:

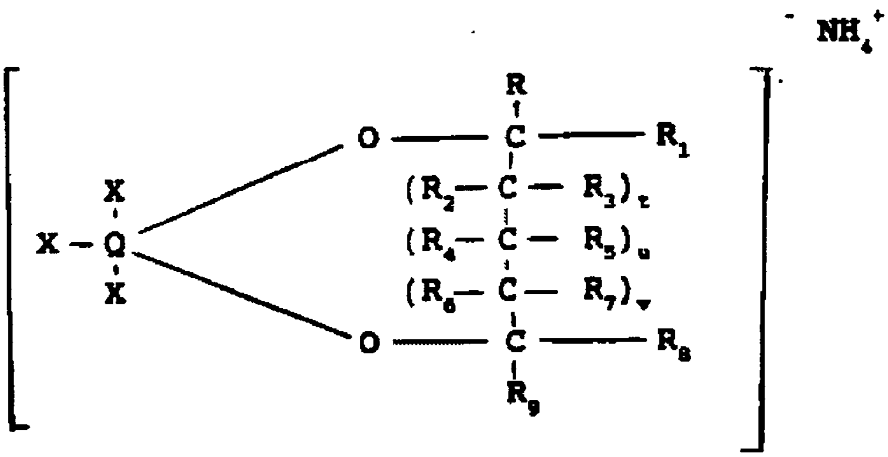

(A)

oder

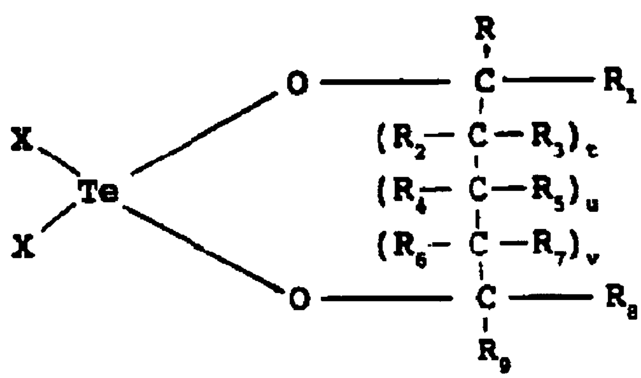

(B)

oder

$$TeX_4 \quad (C)$$

oder

$$TeO_2 \quad (D)$$

oder

$$PhTeCl_3 \quad (E)$$

oder

$$(C_6H_5)^+P(TeCl_3(O_2C_2H_4))^- \quad (F)$$

worin Q entweder Te oder Se ist; t entweder 1 oder 0 ist; u entweder 1 oder 0 ist; v entweder 1 oder 0 ist; R, $R_1$, $R_2$, $R_3$, $R_4$, $R_5$, $R_6$, $R_7$, $R_8$ sowie $R_9$ gleich oder verschieden und unabhängig voneinander ausgewählt sind aus Wasserstoff, Hydroxyalkyl mit 1 bis 6 Kohlenstoffen, Hydroxy, Alkyl mit 1 bis 6 Kohlenstoffatomen, Halogen, Haloalkyl mit 1 bis 6 Kohlenstoffatomen, Carboxy, Alkylcarbonylalkyl mit 2 bis 10 Kohlenstoffen, Alkanoyloxy mit 1 bis 6 Kohlenstoffatomen, Carboxyalkyl mit 1 bis 6 Kohlenstoffatomen, Acyl, Amido, Cyano, Amidoalkyl mit 1 bis 6 Kohlenstoffen, N-Monoalkylamidoalkyl mit 2 bis 10 Kohlenstoffen, N,N-Dialkylamidoalkyl mit 4 bis 10 Kohlenstoffen, Cyanoalkyl mit 1 bis 6 Kohlenstoffen, Alkoxy mit 1 bis 6 Kohlenstoffatomen, Alkoxyalkyl mit 2 bis 10 Kohlenstoffatomen und $-COR_{10}$, wobei $R_{10}$ Alkyl mit 1 bis 6 Kohlenstoffen ist; sowie X ein Halogen ist.

**2.** Zusammensetzung nach Anspruch 1, wobei die Taxan-Verbindung ausgewählt ist aus solchen der Formel:

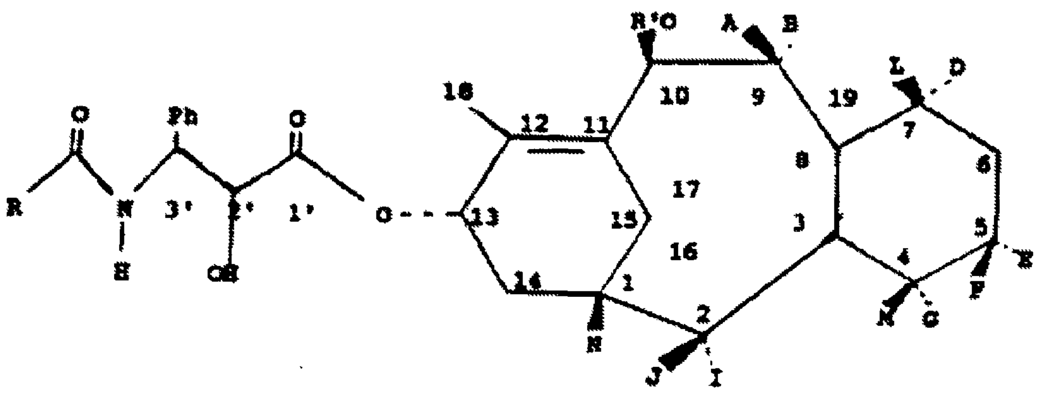

worin
A und B unabhängig voneinander Wasserstoff oder Alkanoyloxy sind oder
A und B zusammen ein Oxo bilden;
L und D unabhängig voneinander Wasserstoff oder Hydroxy sind;
E und F unabhängig voneinander Wasserstoff oder Alkanoyloxy mit 1 bis 6 Kohlenstoffatomen sind;
E und F zusammen ein Oxo bilden;
G und R' Wasserstoff oder Alkanoxy mit 1 bis 6 Kohlenstoffatomen oder Alkanoyloxy mit 1 bis 6 Kohlenstoffatomen oder Aroyloxy sind oder
G und M zusammen ein Oxo oder Methylen bilden oder
G und M zusammen einen Oxocyclopropylring bilden oder
M und F zusammen einen Oxocyclobutylring bilden;

J Wasserstoff oder Aroyloxy oder Alkanoyloxy mit 1 bis 6 Kohlenstoffatomen ist oder
I Wasserstoff oder Aroyloxy ist; oder
I und J zusammen genommen ein Oxo bilden;
N Wasserstoff, Hydroxy oder Alkoxy 1 bis 6 Kohlenstoffatomen ist; und
R Aryl, Alkyl mit 1 bis 6 Kohlenstoffatomen, Alkoxy mit 1 bis 6 Kohlenstoffatomen oder Alkenyl mit 2 bis 6 Kohlenstoffatomen ist;
und die mit den in der Formel als 2' und 3' **gekennzeichneten** Kohlenstoffen verbundenen Hydroxyl- und die Carbonylamido-Substituenten vertauscht sein können.

**3.** Zusammensetzung nach Anspruch 2, wobei die Taxan-Verbindung Paclitaxel, eine Verbindung der Formel, wobei A und B zusammen einen Oxo bilden; L und N Hydroxy sind; D, E und J Wasserstoff sind; G Acetat ist; M und F zusammen einen Oxocyclobutylring bilden; I Benzoyloxy ist und R Phenyl ist, ist.

**4.** Zusammensetzung nach Anspruch 1, wobei die Tellur- oder Selen-Verbindung Ammoniumtrichloro(dioxyethylen-O,O-tellurat), eine Verbindung der Formel (A), wobei Q Tellur ist; R, $R_1$, $R_8$ sowie $R_9$ Wasserstoff sind und t, u sowie v 0 sind, ist.

**5.** Zusammensetzung nach Anspruch 1, wobei die Taxan-Verbindung ungefähr 10 bis ungefähr 90 Gewichtsprozent sowie die Tellur- oder Selen-Verbindung ungefähr 90 bis ungefähr 10 Gewichtsprozent der Kombination ausmachen.

**6.** Pharmazeutische Zusammensetzung, welche eine Taxan-Verbindung sowie eine Tellur- oder Selen-Verbindung enthält, des weiteren kombiniert mit einem oder mehreren pharmazeutisch akzeptablen, inerten oder physiologisch aktiven Verdünnungsmittel oder Hilfsstoff.

## Revendications

**1.** Composition comprenant une combinaison d'un composé de taxane et d'un composé de tellure ou de sélénium en des quantités respectives efficaces pour traiter des tumeurs malignes de façon à produire une augmentation plus que simplement additive de l'effet thérapeutique comparativement au composé de taxane et au composé de tellure ou de sélénium utilisés seuls, ledit composé de tellure ou de sélénium étant choisi parmi les formules :

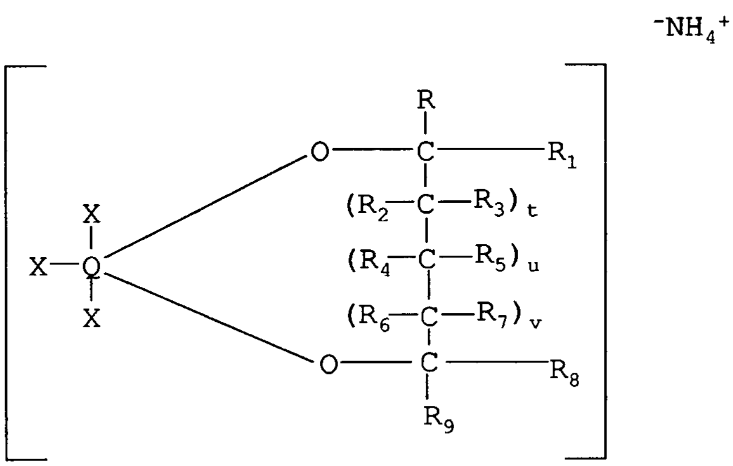

(A)

ou

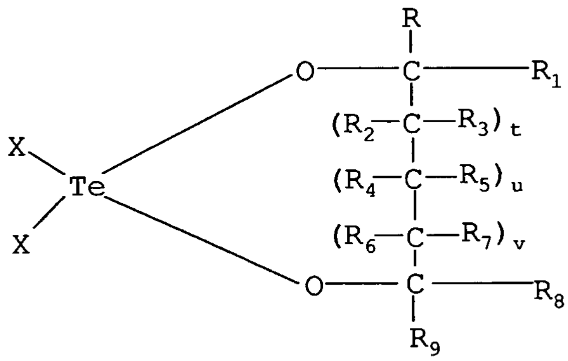

(B)

ou

$$TeX_4 \qquad (C)$$

ou

$$TeO_2 \qquad (D)$$

ou

$$PhTeCl_3 \qquad (E)$$

ou

$$(C_6H_5)\ 'P\ (TeCl_3(O_2C_2H_4))^- \qquad (F)$$

dans lesquelles Q est Te ou Se ; t est égal à 1 ou 0 ; u est égal à 1 ou 0 ; v est égal à 1 ou 0 ; R, $R_1$, $R_2$, $R_3$, $R_4$, $R_5$, $R_6$, $R_7$, $R_8$ et $R_9$ sont identiques ou différents est sont choisis indépendamment parmi l'hydrogène, un radical hydroxyalkyle ayant 1 à 6 carbones, hydroxy, alkyle ayant 1 à 6 atomes de carbone, halogène, halogénoalkyle ayant 1 à 6 atomes de carbone, carboxy, alkylcarbonylalkyle ayant 2 à 10 carbones, alcanoyloxy ayant 1 à 6 atomes de carbone, carboxyalkyle ayant 1 à 6 atomes de carbone, acyle, amido, cyano, amidoalkyle ayant 1 à 6 carbones, N-monoalkylamidoalkyle ayant 2 à 10 carbones, N,N-dialkylamidoalkyle ayant 4 à 10 carbones, cyanoalkyle ayant 1 à 6 carbones, alooxy ayant 1 à 6 atomes de carbone, alcoxyalkyle ayant 2 à 10 atomes de carbone et $-COR_{10}$ où $R_{10}$ est un radical alkyle ayant 1 à 6 carbones ; et X est un halogène.

**2.** Composition telle que définie dans la revendication 1, dans laquelle le composé de taxane est choisi parmi ceux de formule :

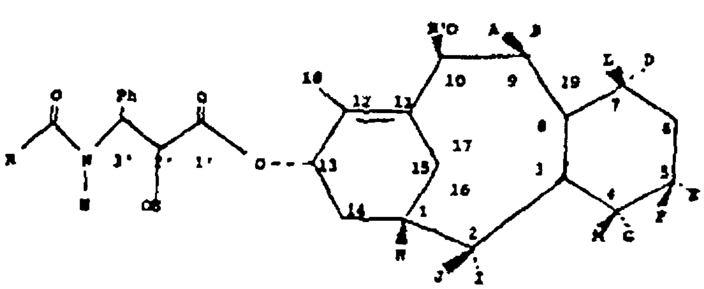

dans laquelle

A et B sont indépendamment l'hydrogène ou un radical alcanoyloxy ou
A et B forment ensemble un radical oxo ;
L et D sont indépendamment l'hydrogène ou un radical hydroxy ;
E et F sont indépendamment l'hydrogène ou un radical alcanoyloxy ayant 1 à 6 atomes de carbone ;
ou E et F forment ensemble un radical oxo ;
G et R' sont l'hydrogène ou un radical alcanoxy ayant 1 à 6 atomes de carbone ou alcanoyloxy ayant 1 à 6 atomes de carbone ou aroyloxy ou
G et M forment ensemble un radical oxo ou méthylène ou
G et M forment ensemble un cycle oxocyclopropyle ou
M et F forment ensemble un cycle oxocyclobutyle ;
J est l'hydrogène ou un radical aroyloxy ou alcanoyloxy ayant 1 à 6 atomes de carbone ; ou
I est l'hydrogène ou un radical aroyloxy ; ou
I et J forment ensemble un radical oxo ;
N est l'hydrogène, un radical hydroxy ou alcoxy ayant 1 à 6 atomes de carbone ; et
R est un radical aryle, alkyle ayant 1 à 6 atomes de carbone, alcoxy ayant 1 à 6 atomes de carbone ou alcényle ayant 2 à 6 atomes de carbone ; et les substituants hydroxyle et carbonylamido fixés aux carbones désignés 2' et 3' dans la formule peuvent être transposés.

**3.** Composition telle que définie dans la revendication 2, dans laquelle le composé de taxane est le paclitaxel, composé de ladite formule dans laquelle A et B forment ensemble un radical oxo ; L et N sont un radical hydroxy, D, E et J sont l'hydrogène, G est l'acétate ; M et F forment ensemble un cycle oxocyclobutyle ; I est un radical benzoyloxy ; et R est un radical phényle.

**4.** Composition telle que définie dans la revendication 1, dans laquelle le composé de tellure ou de sélénium est le trichloro(dioxoéthylène-O,O-tellurate) d'ammonium, composé de formule (A) dans laquelle Q est le tellure ; R, $R_1$, $R_8$ et $R_9$ sont l' hydrogène ; et t, u et v sont égaux à 0.

**5.** Composition telle que définie dans la revendication 1, dans laquelle le composé de taxane représente environ 10 à environ 90 parties en poids et le composé de tellure ou de sélénium représente environ 90 à environ 10 parties en poids de la combinaison.

**6.** Composition pharmaceutique qui contient un composé de taxane et un composé de tellure ou de sélénium, combinée en outre à un ou plusieurs diluants ou adjuvants pharmaceutiquement acceptables inertes ou physiologiquement actifs.

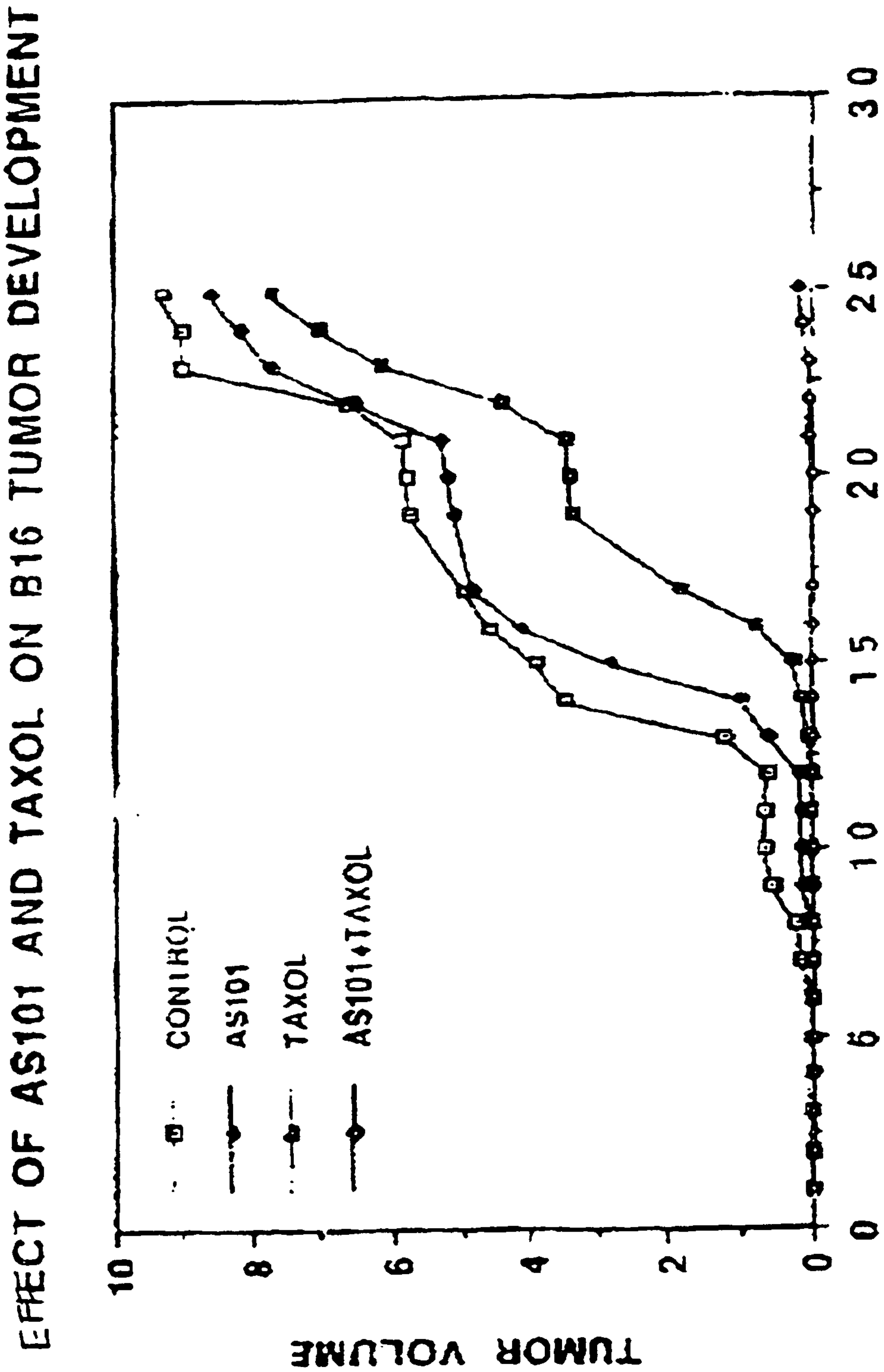
EFFECT OF AS101 AND TAXOL ON B16 TUMOR DEVELOPMENT
TUMOR VOLUME
10
8
6
4
2
0
CONTROL
AS101
TAXOL
AS101+TAXOL
0
5
10
15
20
25
30
DAYS AFTER TUMOR IMPLANTATION